Europäisches Patentamt

European Patent Office

Office européen des brevets

(19)

(11) Publication number: **0 255 025**
**B1**

(12)            # EUROPEAN PATENT SPECIFICATION

(45) Date of publication of the patent specification:
03.10.90

(51) Int. Cl.⁵: **A61M 5/00**

(21) Application number: **87110562.3**

(22) Date of filing: **21.07.87**

(54) Device for coupling a small tube to an apparatus adapted for fitting a syringe to a drug holding bottle.

(30) Priority: **25.07.86 IT 2264786 U**

(43) Date of publication of application:
**03.02.88 Bulletin 88/5**

(45) Publication of the grant of the patent:
**03.10.90 Bulletin 90/40**

(84) Designated Contracting States:
**BE DE FR GB IT LU NL SE**

(56) References cited:
**GB-A- 2 178 664**
**US-A- 4 291 701**
**US-A- 4 369 781**

(73) Proprietor: **FARMITALIA CARLO ERBA S.r.L., Via Carlo Imbonati 24, I-20159 Milano(IT)**

(72) Inventor: **Valentini, Luigi, Via Pergine 12, I-20 1489 Milano(IT)**
Inventor: **Coccia, Mario, Via Gramsci 25, Cesano Boscone (MI)(IT)**

(74) Representative: **Kraus, Walter, Dr. et al, Patentanwälte Kraus, Weisert & Partner Thomas-Wimmer-Ring 15, D-8000 München 22(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a device for coupling a small tube to an apparatus adapted for coupling a syringe to a drug holding bottle, in combination with said apparatus.

US-A 4 291 701 discloses a device for coupling a blood pressure transducer to a catheter to transfer pressure signals from a living organism to the blood pressure transducer. This device has a generally cylindrical body which is provided at one end with a conical cavity closed by a membrane through which the pressure signal are transferred from the cavity to the pressure transducer, and at the other end with a tubular member which protrudes from the body and which is adapted to be coupled to a small tube. This tubular member comprises a port which is connected by a straight passage to the cavity. For connecting the first mentioned end to a circular nut of the blood pressure transducer this end is provided with an externally threaded portion around the cavity, and for connection with the catheter the other end is equipped with an internally threaded fitting surrounding the tubular member. Furthermore, the device has fluted portions or ribs for manual engagement of the device during attachment and removal of the catheter system. For filling the cavity and the passage with a compatible or physiological solution before connecting the device to the catheter system, the port and the passage are adapted to receive a part of a hypodermic needle of a syringe which is simply inserted in the port and passage and prevented from perforating the membrane by a stop within the passage. This device is not suited for coupling, under safe conditions one end of a drug delivering small tube to an apparatus for coupling a syringe to the drug holding bottle.

However, GB-A 2 178 664 which has been published after the priority date of the present patent discloses a device for coupling, under safe conditions, one end of a drug delivering small tube to an apparatus for coupling a syringe to the drug holding bottle.

This device has its free end, that is its end which is to be coupled to the mentioned apparatus, which has substantially the form of a body shaped as the mouth of a conventional bottle of the type used for holding a drug, from the shaped body three or more longitudinal fins projecting, said fins having an even flat longitudinal outer profile and operating for assuring an axial insertion of the mentioned free end of the device and a firm holding of this device in the seat of the apparatus thereon the drug holding syringe is mounted.

As stated in the above mentioned GB-A 2 178 664, the apparatus to be coupled to the syringe is provided with three or more resilient lugs each of which bears a tooth which, as the free end of the device is inserted into said apparatus seat, engages under the device shaped free edge; such an apparatus is disclosed in US-A 4 576 211.

It has been found that the provision of the longitudinal fins projecting from the device shaped body (and the radially outermost longitudinal peripheral edge of which is even) may cause latching problems if the longitudinal fins are arranged in front of the teeth of the apparatus flexible or resilient lugs: in fact if the device is not arranged with its fins disengaged from the apparatus resilient lug teeth, then the device cannot be coupled to the apparatus, and the user may not notice the drawback, with the very dangerous consequence (it should be remembered that many drugs are very toxic) that the device may detach from the apparatus during the drug delivering step.

Thus, the main object of the present invention is to provide a device of the mentioned type which may be easily inserted into the seat of the mentioned apparatus, while holding always a longitudinal orientation during the insertion, and being always firmly held in the seat, in an oscillation free condition, and being firmly engaged by the teeth projecting from the apparatus flexible or resilient lugs, as soon as the device is introduced into said seat.

This object is achieved according to the invention by a device for coupling a small tube to an apparatus adapted for coupling a syringe to a drug holding bottle, in combination with said apparatus, the device comprising a hollow body and a tubular member extending through the hollow body, one end of the tubular body being adapted to be coupled to the small tube and the other end of the tubular body being sealed by a resilient plug; and the apparatus comprising a plurality of resilient lugs extending from one end thereof and surrounding a pad which is penetrable by a needle, each lug being provided with an inwardly projecting tooth; the hollow body comprising a cylindrical collar provided with an annular recess on its outer surface, the arrangement being such that, when the plug is seated on the pad, the legs are in contact with the cylindrical collar and the teeth enter the recess so as to secure the device to the apparatus.

The shape and size of the projecting collar in a perpendicular plane to the longitudinal axis of said shaped body, are analogous to or like those of a conventional bottle of the type used for holding drugs. This collar extends axially peripherally for a length greater than that of the collar of a conventional bottle so as to define an elongated cylindrical surface wherein the continuous annular recess is formed In order to better understand the structure and characteristics of the subject device, a preferred embodiment thereof will be disclosed thereinafter, with reference to the accompanying drawing, where:

Fig. 1 is a perspective view of the device according to the present invention;

Fig. 2 is an enlarged scale cross-sectional view of the device.

The device shown in the drawing comprises a shaped body 1 which is laterally delimited by a cylindrical wall on the outer surface of which there is formed an annular recess 3: inside the cylindrical wall 2 and along its axis, a lug 4 extends, defining a hollow which is open at the top end thereof (as shown in the drawing), while at the other end thereof it is closed by a resilient plug 5 (made of rubber or like material) which is held firmly pressed on the free end of the lug 4 by means of a small rigid holding disc 6 affixed to the cylindrical wall 2.

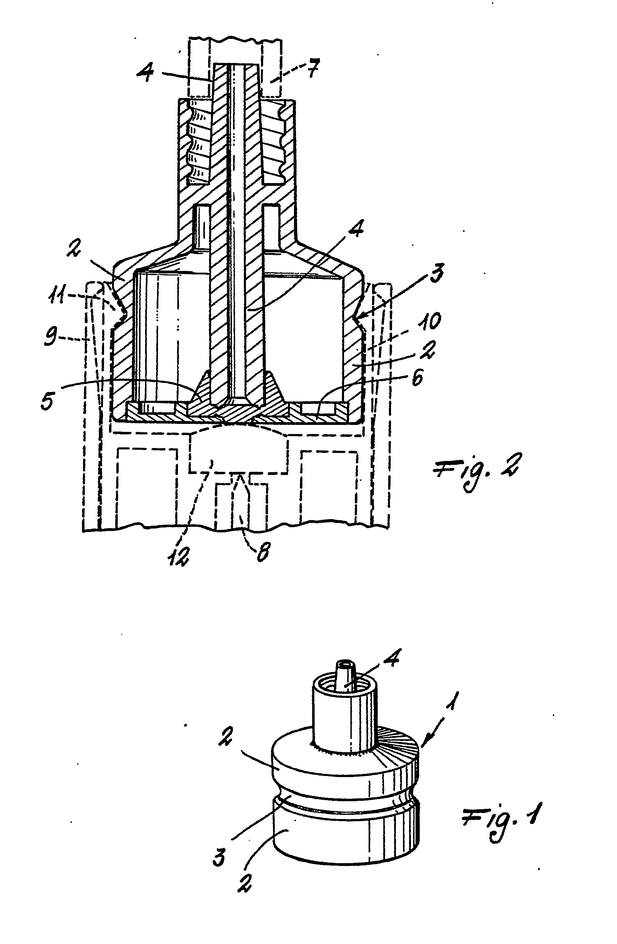

In a known way, on the free projecting end of the hollow lug 4 there is mounted one end of a resilient small tube (a portion of which has been shown by the dashed line in fig.2) to the other end of which an epicranial needle may be coupled for the transfusion into a vein of a patient of a drug which is injected into the hollow of the lug 4 by a needle 8(which is shown in phantom in fig.2) mounted on a not shown syringe.

The needle 8 is included in an apparatus like that disclosed in the US Patent 4,576,211: at the lower portion of fig.2 there is shown,by a dashed line,that end portion of this apparatus thereat a seat is formed for housing and holding the device according to the present invention.

It should briefly be remembered that this seat is defined by a tubular cylindrical wall 9 therewithin resilient lugs 10 extend,a tooth 11 projecting inwardly from each said lug.

At the center of the apparatus seat a rubber or the like pad 12 projects which,as the subject device is locked in said seat,as is schematically shown in fig.2,is held firmly pressed against the resilient plug 5.

The operation of the needle 8 bearing apparatus thereto the subject device may be coupled will be not disclosed herein,since it has been clearly illustrated in the mentioned US Patent 4,576,211.

A main feature of the device according to the present invention is that the cylindrical wall 2 has a cross-section equal or analogous to the cross-section of the collar of a conventional drug holding bottle;that the mentioned cylindrical wall axially extends for a length greater than the depth of the housing seat defined by the cylindrical wall 9 and the resilient lugs 10 of the apparatus, as is clearly shown in fig.2;and that the annular recess 3 is so arranged and shaped that,as the device is coupled to the mentioned apparatus, the teeth 11 of the resilient lugs 10 will always enter said annular recess to be locked therein.

This means that as soon as the device is inserted into the seat of the apparatus and the resilient plug 5 is pressed on the pad 12,the teeth 11 will enter the annular recess 3 thereby always firmly engaging the device with the mentioned apparatus.

The fact that the cylindrical wall 2 axially extends in the indicated way is also an essential feature,since in this way a great resting area is provided for the outer surface of said cylindrical wall against the adjoining surface of the apparatus seat;thus the device will form a nearly rigid body with respect to the apparatus,since it can not oscillate in said apparatus housing seat.

## Claims

A device for coupling a small tube to an apparatus adapted for coupling a syringe to a drug holding bottle, in combination with said apparatus, the device comprising a hollow body (1) and a tubular member (4) extending through the hollow body, one end of the tubular body being adapted to be coupled to the small tube and the other end of the tubular body being sealed by a resilient plug (5); and the apparatus comprising a plurality of resilient lugs (10) extending from one end thereof and surrounding a pad which is penetrable by a needle, each lug being provided with an inwardly projecting tooth (11); the hollow body comprising a cylindrical collar provided with an annular recess (3) on its outer surface, the arrangement being such that, when the plug is seated on the pad, the legs are in contact with the cylindrical collar and the teeth enter the recess so as to secure the device to the apparatus.

## Patentansprüche

1. Vorrichtung zum Ankoppeln eines kleinen Rohrs an eine Einrichtung, die dazu geeignet ist, eine Spritze an eine Arzneimittel enthaltende Flasche anzukoppeln, in Kombination mit dieser Einrichtung, wobei die Vorrichtung einen hohlen Körper (1) und ein rohrförmiges Teil (4), das sich durch den hohlen Körper erstreckt, umfaßt, wobei ein Ende des rohrförmigen Körpers dazu geeignet ist, an das kleine Rohr angekoppelt zu werden, und das andere Ende des rohrförmigen Körpers mittels eines elastischen Stopfens (5) abgedichtet ist; und wobei die Einrichtung eine Mehrzahl von elastischen Ansätzen (10) umfaßt, die sich von einem Ende derselben aus erstrecken und ein Kissen umgeben, das mittels einer Nadel durchdringbar ist, wobei jeder Ansatz mit einem nach einwärts vorstehenden Zahn (11) versehen ist; wobei der hohle Körper einen zylindrischen Bund umfaßt, der auf seiner äußeren Oberfläche mit einer ringförmigen Ausnehmung (3) versehen ist, wobei die Anordnung derart ist, daß dann, wenn der Stopfen auf das Kissen aufgesetzt ist, die Ansätze in Kontakt mit dem zylindrischen Bund sind und die Zähne in die Ausnehmung eintreten, so daß sie die Vorrichtung an der Einrichtung befestigen.

## Revendications

Dispositif pour raccorder un petit tube à un appareil adapté pour relier une seringue à un flacon contenant un médicament, en combinaison avec ledit appareil, dispositif qui comporte un corps creux (1) et un organe tubulaire (4) qui s'étend à travers le corps creux, l'une des extrémités du corps tubulaire étant adaptée pour être raccordée au petit tube, tandis que son autre extrémité est fermée hermétiquement par un bouchon élastique (5); l'appareil comportant plusieurs protubérances élastiques (10) qui s'étendent depuis l'une de ses extrémités et entourent un tampon apte à être traversé par une aiguille, protubérances qui présentent respectivement une dent (11) faisant saillie vers l'intérieur; tandis que le corps creux comporte un épaulement cylindrique muni d'un renfoncement annulaire (3) sur sa surface extérieure, la conception étant telle que, lorsque le bouchon est en appui sur le tampon, les protubérances sont en contact avec l'épaulement cylindrique, les dents pénétrant dans le renfoncement de façon à assujettir le dispositif à l'appareil.

4    7

2    4

11    3

9    10

5    2

   6

12    8

*Fig. 2*

4

2

1

3

2

*Fig. 1*